# EUROPÄISCHE PATENTSCHRIFT

(11) **EP 1 523 932 B1**
(45) Veröffentlichungstag und Bekanntmachung des Hinweises auf die Patenterteilung: **03.05.2006**
(21) Anmeldenummer: 03023645.9
(22) Anmeldetag: 17.10.2003
(51) Int. Cl.: A61B 1/00

(54) **Endoskop**
Endoscope
Endoscope

(43) Veröffentlichungstag der Anmeldung: 20.04.2005
(73) Patentinhaber: Henke-Sass, Wolf GmbH, D-78532 Tuttlingen (DE); EKL Medical Ltd., Oldham OL4 1DE (GB)
(72) Erfinder: Kiehn, Ralf, 78606 Seitingen-Oberflacht (DE); Rehe, Oliver Heiko, 78532 Tuttlingen (DE); Weller, Thomas Paul, 78532 Tuttlingen (DE); Knight, Martin, Dr., c/o EKL Medical Ltd., Hamilton Street, Oldham OL4 1DE (GB)
(74) Vertreter: Geyer, Fehners & Partner

(56) Entgegenhaltungen:
- US-A- 2 038 393
- US-A- 3 835 842
- US-A- 5 807 240
- US-A1- 2002 058 859
- US-B1- 6 358 200

## Beschreibung

Die Erfindung betrifft ein Endoskop, das insbesondere im medizinischen Bereich eingesetzt wird. Ein solches Endoskop gemäß dem Oberbegriff des Anspruchs 1 wird beispielsweise in US-A-6358200 beschrieben.

Solche Endoskope müssen, wenn beispielsweise an der Wirbelsäule operiert wird, möglichst klein und gut sterilisierbar sein.

Es ist daher Aufgabe der Erfindung, ein Endoskop bereitzustellen, das einen Schaft mit möglichst geringem Durchmesser und gleichzeitig mehrere Funktionalitäten aufweist.

Erfindungsgemäß wird die Aufgabe gelöst durch ein Endoskop mit einem ein distales und ein proximales Ende aufweisenden Schaft, der ein äußeres Rohr aufweist, in dem ein inneres Rohr derart eingesetzt ist, daß es gegenüber dem äußeren Rohr verdrehbar sowie in Längsrichtung der Rohre verschiebbar ist und daß zwischen den beiden Rohren ein erster Kanal ausgebildet ist, und ferner mit einem mit dem proximalen Ende des Schafts verbundenen Hauptteil, wobei in dem inneren Rohr ein Instrumentenrohr sowie eine Bildaufnahmeoptik angeordnet sind und ein zweiter Kanal ausgebildet ist und wobei sowohl das Instrumentenrohr als auch die Bildaufnahmeoptik drehfest mit dem inneren Rohr verbunden sind.

Da das Instrumentenrohr und die Bildaufnahmeoptik drehfest mit dem inneren Rohr verbunden sind, können das Instrumentenrohr und die Bildaufnahmeoptik gegenüber dem äußeren Rohr verdreht werden, was insbesondere bei der Verwendung des Endoskops im medizinischen Bereich von Vorteil ist. Für die Verdrehbarkeit des Instrumentenrohrs und der Bildaufnahmeoptik ist es bei dem erfindungsgemäßen Endoskop jedoch nicht notwendig, daß die Bildaufnahmeoptik und das Instrumentenrohr gegenüber dem inneren Rohr verdrehbar angeordnet sind. Damit wird es vorteilhaft möglich, die Querschnittsformen und Größen des Instrumentenrohrs und der Bildaufnahmeoptik relativ frei zu wählen.

So kann mit dem erfindungsgemäßen Endoskop der Bereich am distalen Ende mittels der Bildaufnahmeoptik betrachtet werden und gleichzeitig kann mittels einem im Instrumentenrohr angeordneten Instrument in diesem Bereich manipuliert werden. Ferner können die zwei Kanäle noch als Spül- und Absaugkanal eingesetzt werden. Somit sind verschiedene Funktionalitäten beim erfindungsgemäßen Endoskop verwirklicht.

Bevorzugt sind bei dem erfindungsgemäßen Endoskop sowohl das Instrumentenrohr als auch die Bildaufnahmeoptik so mit dem inneren Rohr verbunden, daß sie in Längsrichtung nicht gegenüber dem inneren Rohr verschiebbar sind.

Das Instrumentenrohr, die Bildaufnahmeoptik und das innere Rohr bilden somit eine Einheit, die gegenüber dem äußeren Rohr drehbar und in der Längsrichtung verschiebbar ist.

Durch das Vorsehen von zwei voneinander getrennten Kanälen im Schaft des Endoskops kann einer der beiden Kanäle als Spülkanal, über den eine Spülflüssigkeit dem am distalen Ende des Schafts liegenden Bereich zugeführt werden kann, und der andere Kanal als Absaugkanal eingesetzt werden, über den die Spülflüssigkeit wiederum am distalen Ende des Schafts abgesaugt wird.

Insbesondere kann das äußere Rohr am distalen offen ausgebildet sein, und in einer Seitenansicht gesehen, relativ zur Längsrichtung des Schafts abgeschrägt ausgebildet sein. Dadurch wird das distale Ende selbst auch gleich als Instrument einsetzbar, was insbesondere bei Operationen an der Wirbelsäule als Werkzeug zum zur Seiteschieben von Nerven oder auch als Schutz der Nerven gegenüber einem Instrument, das durch das Instrumentenrohr am distalen Ende im zu operierenden Bereich eingesetzt wird, nützlich ist.

Insbesondere ist sowohl das Instrumentenrohr als auch die Bildaufnahmeoptik lösbar mit dem inneren Rohr verbunden. Dies erleichtert die Sterilisation und insbesondere das Autoklavieren des Endoskops. In dieser Hinsicht kann das Endoskop ferner derart weitergebildet werden, daß auch das äußere Rohr vollständig von dem inneren Rohr abgezogen werden kann, so daß auch das äußere Rohr und das innere Rohr separat sterilisiert werden können.

Ferner kann beim erfindungsgemäßen Endoskop am proximalen Ende des Schafts bzw. des äußeren Rohrs ein erstes Absperrventil angeordnet sein, das mit dem ersten Kanal in Verbindung steht. Dieses Absperrventil ist bevorzugt um die Längsrichtung bzw. um eine Achse parallel zur Längsrichtung drehbar angeordnet. Damit läßt sich das Absperrventil in eine gewünschte Stellung relativ zum Schaft bringen.

Ferner kann am proximalen Ende des Schafts bzw. des inneren Rohrs ein zweites Absperrventil angeordnet sein, das mit dem zweiten Kanal in Verbindung steht. Bevorzugt ist auch das zweite Abspenventil so angeordnet, daß es um die Längsrichtung bzw. um eine Achse parallel zur Längsrichtung drehbar ist. Damit ist auch das zweite Absperrventil relativ zum Schaft relativ frei positionierbar.

Die Kombination der Absperrventile mit dem Merkmal, daß das Instrumentenrohr und die Bildaufnahmeoptik lösbar mit dem inneren Rohr verbunden sind, bringt den Vorteil mit sich, daß das Instrumentenrohr und die Bildaufnahmeoptik ausgewechselt werden können, ohne daß mit den Absperrventilen verbundene Schläuche entfernt und wieder angebracht werden müssen. Dies führt insbesondere bei medizinischer Anwendung zu einer verbesserten Ergonomie.

Besonders bevorzugt ist es, wenn das innere Rohr einen ovalen Querschnitt aufweist. Dadurch läßt sich einerseits die Verdrehbarkeit des inneres Rohrs gegenüber dem äußeren Rohr sicherstellen und andererseits ist der erste Kanal zwischen dem äußeren Rohr und inneren Rohr vorgesehen.

Ferner kann bei dem erfindungsgemäßen Endoskop das innere Rohr am distalen Ende offen und, in einer Seitenansicht, relativ zur Längsrichtung abgeschrägt ausgebildet sein.

Besonders bevorzugt ist es bei dem erfindungsgemäßen Endoskop, wenn das Instrumentenrohr und die Bildaufnahmeoptik innerhalb eines Endoskoprohrs angeordnet sind, das seinerseits in das innere Rohr derart eingesetzt ist, daß zwischen dem inneren Rohr und dem Endoskoprohr der zweite Kanal ausgebildet ist. Durch diese Ausbildung wird die gesamte Anordnung der Rohre sehr platzsparend realisiert.

Besonders vorteilhaft ist es dann, wenn das Endoskoprohr einen ovalen Querschnitt aufweist, der so gewählt ist, daß im Querschnitt gesehen, die maximale lichte Weite im Bereich des Instrumentenrohrs größer ist als die maximale lichte Weite im Bereich der Bildaufnahmeoptik. Dadurch kann ein Instrumentenrohr mit einer größeren Querschnittsfläche als die Bildaufnahmeoptik vorgesehen werden, wodurch vorteilhaft der Platz für die Instrumente größer wird.

Bei einer bevorzugten Ausgestaltung des erfindungsgemäßen Endoskops ist am proximalen Ende des Instrumentenrohrs ein Dichtsystem ausgebildet, das das proximale Ende des Instrumentenrohrs unabhängig davon abdichtet, ob ein Instrumentenrohr eingeführt ist oder nicht.

Dazu kann das Dichtsystem zwei Dichteinheiten aufweisen, wobei die erste Dichteinheit für die Abdichtung sorgt, wenn ein Instrument eingesetzt ist, und die zweite Dichteinheit für die Abdichtung sorgt, wenn kein Instrument eingesetzt ist. Die zwei Dichteinheiten sind bevorzugt, in Längsrichtung des Instrumentenrohrs gesehen, hintereinander angeordnet.

Insbesondere kann das erfindungsgemäße Endoskop so weitergebildet werden, daß das Instrumentenrohr und die Bildaufnahmeoptik miteinander verbunden sind und eine erste Endoskopeinheit bilden, und daß eine zweite Endoskopeinheit mit einem weiteren Instrumentenrohr und einer weiteren Bildaufnahmeoptik vorgesehen ist, wobei die Endoskopeinheiten abwechselnd in das innere Rohr einsetzbar und im eingesetzten Zustand mit dem inneren Rohr verbindbar sind. Damit wird ein Endoskop bzw. Endoskopsystem bereitgestellt, bei dem die Endoskopeinheiten leicht austauschbar sind. Da die eingesetzten Endoskopeinheiten austauschbar ausgebildet sind, kann der Austausch während des bestimmungsgemäßen Gebrauchs des Endoskops durchgeführt werden (beispielsweise während einer Operation).

Die zweite Endoskopeinheit kann in gleicher Weise wie die oben beschriebene erste Endoskopeinheit weitergebildet werden. Die beiden Endoskopeinheiten können sich insbesondere in einem Merkmal unterscheiden. Dies kann beispielsweise die Blickrichtung der Bildaufnahmeoptik bezogen auf die Längsrichtung sein.

Natürlich kann das Endoskopsystem mehr als zwei Endoskopeinheiten aufweisen, die sich bevorzugt zumindest in einem Merkmal (z. B. die Blickrichtung der Bildaufnahmeoptik) unterscheiden.

Für die Verbindung zwischen der entsprechenden Endoskopeinheit und dem inneren Rohr kann eine Verriegelungseinheit am Hauptteil (bevorzugt am proximalen Ende des Hauptteils) ausgebildet sein, mit der die gewünschte Verbindung realisierbar und auch wieder lösbar ist.

Die Erfindung wird nachfolgend unter Bezugnahme auf die Zeichnungen beispielshalber noch näher erläutert. Von den Zeichnungen zeigen:
- Fig. 1: eine erste Ausführungsform des erfindungsgemäßen Endoskops in einer ersten Betriebsstellung,
- Fig. 2: das Endoskop von Fig. 1 in einer zweiten Betriebsstellung,
- Fig. 3: den Schnitt A-A des Schafts von Fig. 1,
- Fig. 4.: eine Ansicht der Endoskopeinheit des Endoskops von Fig. 1 und 2, und
- Fig. 5: eine vergrößerte Schnittdarstellung des Dichtsystems am proximalen Ende des Endoskops von Fig. 1 und 2,
- Fig. 6: eine Darstellung einer Kreuzschlitzdichtung des Dichtsystems von Fig. 5 und
- Fig. 7: eine vergrößerte Schnittdarstellung der Schnellverriegelungseinheit des in Fig. 1 und 2 gezeigten Endoskops

Das Endoskop umfaßt einen Schaft 1 mit einem distalen Ende 2 und einem proximalen Ende 3 sowie ein Hauptteil 4.

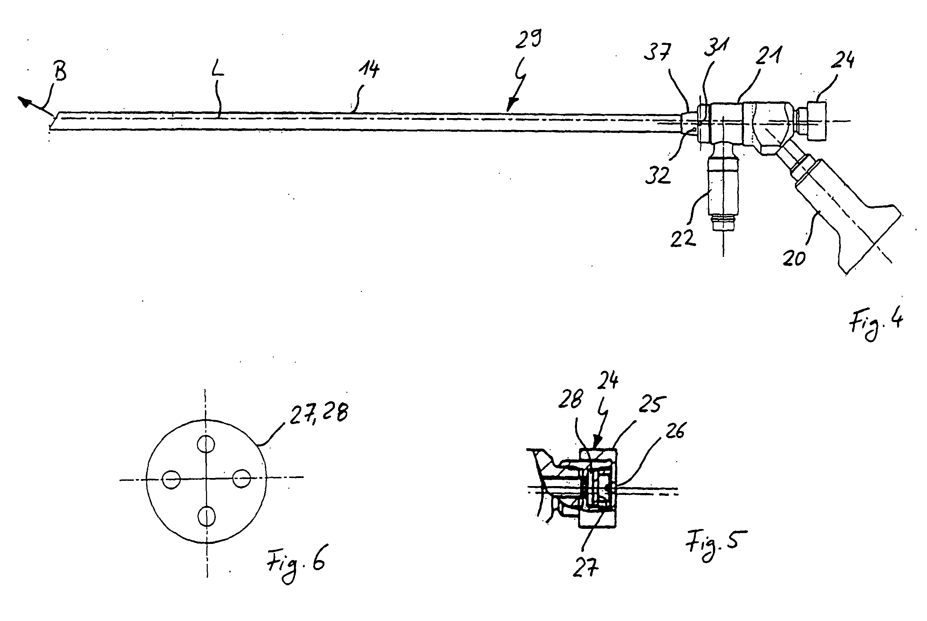

Der Schaft 1 weist ein äußeres Rohr 5 mit einer Länge von ca. 16 cm mit kreisrundem Querschnitt mit einem Außendurchmesser von ca. 7,5 mm auf, in das ein inneres Rohr 6 mit ovalem Querschnitt eingesetzt ist. Der Querschnitt des inneres Rohrs 6 ist so gewählt, daß im eingesetzten Zustand, wie am besten aus Fig. 3 ersichtlich ist, zwischen dem inneren und äußeren Rohr 6, 5 ein erster Kanal 7, der hier in Absaugkanal ist, ausgebildet ist. Am proximalen Ende 8 des äußeres Rohrs 5 ist ein mit dem äußeren Rohr 5 verbundenes Griffstück 9 angeordnet, das ein Absperrventil 10 mit einem Anschlußstutzen 11 (z. B. Luer Lock) trägt. Mittels dem Absperrventil 10 kann eine Fluidverbindung zwischen dem Anschlußstutzen 11 und dem ersten Kanal 7 hergestellt oder unterbrochen werden. Das Absperrventil 10 (zusammen mit dem Anschlußstutzen 11) ist so mit dem Griffstück 9 verbunden, daß es um die Längsachse L des Schafts 1 drehbar ist.

An dem Hauptteil 4 ist eine Hülse 12 befestigt, in die das proximale Ende des inneres Rohrs 6 eingesetzt ist. Die Befestigung des inneren Rohrs 6 in der Hülse 12 kann mittels Löten oder Schweißen hergestellt sein. Alternativ ist es möglich, das Hauptteil 4 und die Hülse 12 einstückig auszubilden. Auf der Außenseite der Hülse 12 ist das Griffstück 9 in Längsrichtung L verschiebbar und drehbar gelagert, so daß das äußere Rohr 5 gegenüber dem inneren Rohr 6 verschiebbar und drehbar ist. Die Hülse 12 weist eine Ringdichtung 13 auf, die neben der gewünschten Abdichtung des proximalen Endes des ersten Kanals 7 auch gleichzeitig noch dazu dient, die notwendige Kraft vorzugeben, die benötigt wird, um das äußere Rohr 5 gegenüber dem inneren Rohr 6 in Längsrichtung des Schafts 1 zu verschieben und/oder zu drehen.

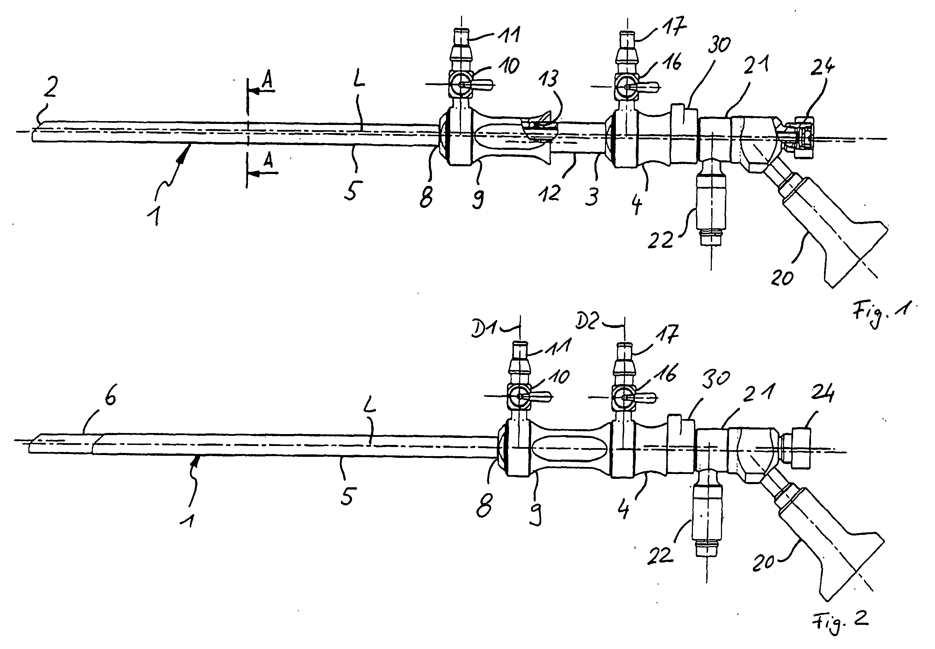

In Fig. 1 ist das Endoskop in einer ersten Endstellung gezeigt, in der die distalen Enden des inneren und äußeren Rohrs 6, 5 auf gleicher Höhe sind. In Fig. 2 ist eine zweite Endstellung gezeigt, in der das distale Ende des inneren Rohrs 6 aus dem äußeren Rohr 5 vorsteht.

Durch die Verwendung der Hülse 12, ist es möglich, ein dünnwandiges inneres Rohr 6 mit kleiner Querschnittsfläche für den Schaft 1 bereitzustellen, was insbesondere bei medizinischen Anwendungen (beispielsweise Operationen der Wirbelsäule) von Vorteil ist. Für die notwendige Stabilität zur Führung des Griffstücks 9 bei der Längsverschiebung und/oder der Drehung des äußeren Rohrs 5 gegenüber dem inneren Rohr 6 sorgt dann die Hülse 12, die dickwandiger ausgebildet ist.

Wie am bestens aus Fig. 3 ersichtlich ist, ist in dem inneren Rohr 6 ein Endoskoprohr 14 mit ovalem Querschnitt eingesetzt. Der Querschnitt des Endoskoprohrs 14 ist dabei so gewählt, daß zwischen dem inneren Rohr 6 und dem Endoskoprohr 14 ein zweiter Kanal 15 ausgebildet ist. Am Hauptteil 4 ist für den zweiten Kanal 15 ein Absperrventil 16 mit einem Anschlußstutzen 17 angeordnet. Mittels dem Absperrventil 16 kann eine Fluidverbindung zwischen dem zweiten Kanal 15 und dem Anschlußstutzen 17 hergestellt und unterbrochen werden. In dem hier beschriebenen Ausführungsbeispiel dient der zweite Kanal 15 als Spülkanal. Das Absperrventil 16 und der Anschlußstutzen 17 sind so am Hauptteil 4 angeordnet, daß sie um die Längsachse L des Schafts 1 drehbar sind.

Innerhalb des Endoskoprohrs 14 ist ein Instrumentenrohr 18 mit kreisrundem Querschnitt und ein Optikrohr 19 mit kreisrundem Querschnitt angeordnet, wobei der Durchmesser des Optikrohrs 19 kleiner ist als der des Instrumentenrohrs (hier 2,3 mm gegenüber 3,7 mm). Die ovale Form des Endoskoprohrs 14 ist so gewählt, daß die maximale lichte Weite W1 im Bereich des Instrumentenrohrs 18 im wesentlichen dem Außendurchmesser des Instrumentenrohrs 18 entspricht und die maximale lichte Weite W2 im Bereich des Optikrohrs 19 im wesentlichen dem Außendurchmesser des Optikrohrs 19 entspricht. Damit weist das Endoskoprohr 14, wie in Fig. 3 ersichtlich ist, einen im wesentlichen eiförmigen Querschnitt auf.

Innerhalb des Endoskoprohrs 14 und zwischen dem Instrumentenrohr 18 und dem Optikrohr 19 sind noch Lichtleitfasern (nicht eingezeichnet) angeordnet, die zur Beleuchtung des aufzunehmenden Objektes dienen. In dem Optikrohr 19 ist eine aus optischen Endoskopen bekannte Linsenoptik angeordnet, die das aufgenommene Bild bis zu einem am proximalen Ende des Endoskops angeordneten Augenstücks 20 überträgt. Natürlich kann anstatt der Linsenoptik beispielsweise am distalen Ende des Optikrohrs 19 ein elektronischer Bildsensor mit, falls notwendig, vorgeschalteter Optik, vorgesehen sein, der dann die Bildsignale über eine elektronische Leitung zum Augenstück 20, das dann eine entsprechende Bildanzeigeeinheit aufweisen kann, überträgt.

Das Optikrohr 19, das Instrumentenrohr 18 und das Endoskoprohr 14 sind am proximalen Ende des Endoskoprohrs 14 mit einem Endstück 21 fest verbunden (beispielsweise verlötet oder verschweißt). An dem Endstück 21 ist ein Anschlußstutzen 22 befestigt, über den die Lichtleitfasem mit Licht beaufschlagbar sind. Ferner ist das Augenstück 20, über das entweder direkt das aufgenommene Bild betrachtet werden kann oder an das eine Kamera (nicht gezeigt) anschließbar ist, mit dem Endstück 21 lösbar verbunden. Dies bringt den Vorteil mit sich, daß, wenn am Augenstück 20 eine Kamera mit einer sterilen Abdeckung befestigt ist, wie dies bei Operationen vorkommen kann, bei Wechsel des Endoskoprohrs 14 das Augenstück 20 von dem Endoskoprohr gelöst werden (zusammen mit der Kamera) und dann an dem neuen Endoskoprohr gleich wieder befestigt werden kann.

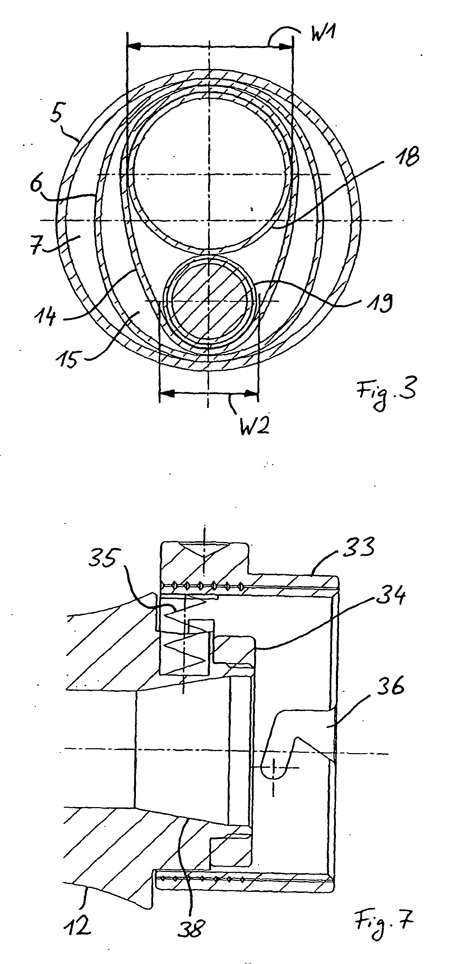

Das Instrumentenrohr 18 verläuft durch das Endstück 21 hindurch und ist mit einem Dichtsystem 24 so abgedichtet, daß das proximale Ende des Instrumentenrohrs 18 abgedichtet ist, unabhängig davon ob ein Instrument eingeführt oder nicht. Dazu weist das Dichtsystem 24, wie am besten aus Fig. 5 ersichtlich ist, eine Gummi-Dichtkappe 25, mit einem zentralen Loch 26, dessen Größe so gewählt ist, daß bei einem eingeführten Instrument die Abdichtung mittels der Dichtkappe 25 erfolgt, sowie beispielsweise zwei Silikon-Kreuzschlitzdichtungen 27 und 28 (Fig. 6) auf, die im eingebauten Zustand so gegeneinander verdreht sind, daß sie dichtend in dem Fall wirken, in dem kein Instrument eingesetzt ist.

Das Endoskoprohr 14, das Instrumentenrohr 18, das Optikrohr 19, die Lichtleitfasem und das Endstück 21 mit dem Anschlußstutzen 22, dem Augenstück 20 und dem Dichtsystem 24 bilden eine Endoskopeinheit 29. Die beschriebene Endoskopeinheit 29 ist eine sogenannte 30°-Einheit, da die Aufnahmerichtung B, wie in Fig. 4 schematisch eingezeichnet ist, gegenüber der Längsrichtung um 30° geneigt ist. Der Schaft 1 des Endoskops weist das äußere Rohr 5, das innere Rohr 6, die Hülse 12 und die entsprechenden Teile der darin eingesetzten Endoskopeinheit 29 auf.

Um nun beispielsweise während einer Operation einen schnellen Austausch der Endoskopeinheit 29 durchführen zu können (ohne das gesamte Endoskop aus dem zu operierenden Bereich entfernen zu müssen), da der Operateur z. B. eine 0°-Endoskopeinheit benötigt (Aufnahmerichtung entlang der Längsrichtung), ist die Endoskopeinheit 29 mittels einer am Hauptteil 4 angeordneten Schnellverriegelungseinheit 30 mit dem inneren Rohr 6 so verbindbar, daß das Endoskoprohr 14 mit dem inneren Rohr 6 drehfest verbunden ist und eine Verschiebung in Längsrichtung zwischen den beiden Rohren 14 und 6 nicht möglich ist.

Es wird zuerst das Einsetzen der Endoskopeinheit 29 beschrieben. Das Endstück 21 weist zwei Verriegelungsstifte 31 (von denen in Fig. 4 nur einer sichtbar ist) sowie ein Führungsstift 32 auf, wie am besten aus Fig. 4 ersichtlich ist. Die Schnellverriegelungseinheit 30 weist, wie am besten aus Fig. 7 ersichtlich ist, ein Griffstück 33 auf, das mit einer Mutter 34 an der Hülse 12 so befestigt ist, daß es in axialer Richtung fixiert ist. In der radialen Richtung, die in Fig. 7 gezeigt ist, ist das Griffstück 33 verschiebbar, wobei es mittels einer zwischen der Hülse 12 und dem Griffstück 33 angeordneten Feder 35 in die gezeigte Verriegelungsposition bewegt ist. Das Griffstück 33 kann, in Fig. 7 gesehen, nach unten gegen die Federkraft gedrückt und dadurch in die Entriegelungsposition bewegt werden. Am proximalen Ende des Griffstücks 33 ist eine bzw. zwei Ausfräsungen 36 (von denen in Fig. 7 nur einer sichtbar ist) für die Verriegelungsstifte 31 sowie eine Längsnut (nicht gezeigt) für den Führungsstift 32 vorgesehen.

Zum Verbinden des Endoskoprohrs 14 mit dem inneren Rohr 6 wird das Endoskoprohr 14 von der proximalen Seite in das innere Rohr 6 eingeschoben, wobei dabei der Führungsstift 31 in die Längsnut der Schnellverriegelungseinheit 30 eingreift. Die Form der Ausfräsung 36 ist so gewählt, daß beim Einschieben des Endoskoprohrs 14 der Verriegelungsstift 31 das Griffstück 33, in Fig. 7 gesehen, nach unten gegen die Federkraft der Feder 35 drückt. Wenn das Endoskoprohr 14 weiter eingeschoben wird, kommt der Verriegelungsstift 31 in einen Verriegelungsabschnitt der Ausfräsung 36, so daß aufgrund der Rückstellkraft der Feder 35 das Griffstück 33 wieder nach oben gedrückt wird und damit eine Verriegelung des Endoskoprohrs 14 gegenüber dem inneren Rohr 6 in Längsrichtung des Schaftes 1 bewirkt. Der in der Nut eingreifende Führungsstift 31 führt dazu, daß die Verbindung drehfest ist, so daß das Endoskoprohr 14 drehfest und nicht längsverschieblich mit dem inneren Rohr 6 verbunden ist. Damit ändert sich bei Bewegung des inneren Rohrs 6 gegenüber dem äußeren Rohr 5 der Abstand zwischen dem Absperrventil 16 und dem Anschlußstutzen 22 und dem Augenstück 20 nicht. Bei der so erzeugten Verbindung liegt ein konischer Abschnitt 37 der Endoskopeinheit 29 dichtend an der konischen Dichtfläche 38 der Hülse so an, daß das proximale Ende des zweiten Kanals 15 abgedichtet ist.

Zum Lösen der Endoskopeinheit 29 muß lediglich das Griffstück 33 nach unten gegen die Feder 35 gedrückt werden und danach das Endoskoprohr 14 an seinem Endstück 21 nach rechts (in Fig. 1 und 2) aus dem inneren Rohr 6 herausgezogen werden.

Um nun den beschriebenen Austausch durchzuführen, wird das Griffstück nach unten gedrückt und die Endoskopeinheit 29 herausgezogen. Sofern noch ein Instrument im Instrumentenrohr 18 ist, wird dies bevorzugt vor dem Herausziehen der Endoskopeinheit 29 entfernt. Falls an dem Augenstück 20 eine Kamera befestigt ist, kann diese ebenfalls vor dem Herausziehen der Endoskopeinheit 29 von dem Augenstück oder zusammen mit dem Augenstück (je nach Bedarf) von der Endoskopeinheit getrennt werden. Danach wird die 0°-Endoskopeinheit in der beschriebenen Weise eingesetzt und mittels der Schnellverriegelungseinheit 30 fixiert. Darauf kann dann das Instrument in den Instrumentenkanal eingeführt und die Kamera (falls gewünscht) am Endstück befestigt werden. Natürlich können auch Endoskopeinheiten mit anderen Neigungen der Aufnahmerichtung verwendet werden, z. B. 45° und 70°. Ein besonderer Vorteil bei diesem Austausch besteht darin, daß mit den Anschlußstutzen 11, 17 verbundene Schläuche nicht entfernt und wieder angebracht werden müssen, so daß der Austausch schnell durchführbar ist.

Das Endoskop mit der Endoskopeinheit 29 und der weiteren 0°-Endoskopeinheit bildet ein Endoskopsystem, bei dem während der Verwendung des Endoskops ein Austausch der Endoskopeinheit leicht durchführbar ist. Dies ist aufgrund des modularen Aufbaus des Endoskops möglich.

Das distale Ende 2 des äußeren Rohrs 5 ist in der Seitenansicht von Fig. 1 und 2 gesehen, abgeschrägt ausgebildet. Damit kann das distale Ende bei Operationen auch gleich als Instrument verwendet werden. In gleicher Weise ist das distale Ende des inneren Rohrs 6 auch abgeschrägt ausgebildet.

Das Endoskop ist so ausgebildet, daß es vollständig zerlegt werden kann. So kann das äußere Rohr 5 zusammen mit Griffstück 9 vollkommen von dem inneren Rohr 6 abgezogen werden. Auch das Endoskoprohr 14 kann, wie beschrieben, von dem inneren Rohr 6 getrennt werden. Damit können die Rohre voneinander getrennt gereinigt und sterilisiert werden. Insbesondere ist dies beim Autoklavieren von Vorteil.

Um einen sichereren Griff am Griffstück zu ermöglichen, weist das Griffstück vier flache Seiten auf, wie in den Darstellungen von Fig. 1 und 2 angedeutet ist.

## Patentansprüche

1. Endoskop mit einem ein distales und ein proximales Ende (2, 3) aufweisenden Schaft (1), der ein äußeres Rohr (5) aufweist, in dem ein inneres Rohr (6) derart eingesetzt ist, daß es gegenüber dem äußeren Rohr (5) verdrehbar ist und daß zwischen den beiden Rohren (5, 6) ein erster Kanal (7) ausgebildet ist, wobei in dem inneren Rohr (6) eine Bildaufnahmeoptik (19) angeordnet ist und ein zweiter Kanal (15) ausgebildet ist und wobei die Bildaufnahmeoptik (19) drehfest mit dem inneren Rohr (6) verbunden ist, **dadurch** charakterisiert, dass das innere Rohr (6) gegenüber dem äußeren Rohr in Längsrichtung der Rohre (5, 6) verschiebbar ist und dass in dem inneren Rohr drehfest ein Instrumentenrohr (18) angeordnet ist.

2. Endoskop nach Anspruch 1, bei dem sowohl das Instrumentenrohr (18) als auch die Bildaufnahmeoptik (19) so mit dem inneren Rohr (6) verbunden sind, daß sie in Längsrichtung nicht gegenüber dem inneren Rohr (6) verschiebbar sind.

3. Endoskop nach einem der obigen Ansprüche, bei dem das äußere Rohr (5) am distalen Ende (2) offen ist und, in einer Seitenansicht gesehen, relativ zur Längsrichtung (L) des Schafts (1) abgeschrägt ausgebildet ist.

4. Endoskop nach einem der obigen Ansprüche, bei dem sowohl das Instrumentenrohr (18) als auch die Bildaufnahmeoptik (19) lösbar mit dem inneren Rohr (6) verbunden sind.

5. Endoskop nach einem der obigen Ansprüche, bei dem am proximalen Ende (3) des Schafts (1) ein erstes Absperrventil (10) angeordnet ist, das mit dem ersten Kanal (7) in Verbindung steht und das bevorzugt drehbar um die Längsrichtung (L) oder um eine Achse parallel zur Längsrichtung (L) angeordnet und ferner bevorzugt so mit dem äußeren Rohr (5) verbunden ist, daß es in Längsrichtung nicht gegenüber dem äußeren Rohr (5) verschiebbar ist.

6. Endoskop nach einem der obigen Ansprüche, bei dem am proximalen Ende des Schafts (1) ein zweites Absperrventil (16) angeordnet, das mit dem zweiten Kanal (15) in Verbindung steht und das bevorzugt drehbar um die Längsrichtung (L) oder um eine Achse parallel zur Längsrichtung (L) angeordnet und ferner bevorzugt so mit dem inneren Rohr (6) verbunden ist, daß es in Längsrichtung nicht gegenüber dem inneren Rohr (6) verschiebbar ist.

7. Endoskop nach einem der obigen Ansprüche, bei dem das innere Rohr (6) einen ovalen Querschnitt aufweist.

8. Endoskop nach einem der obigen Ansprüche, bei dem das innere Rohr (6) am distalen Ende offen ist und, in einer Seitenansicht gesehen, relativ zur Längsrichtung abgeschrägt ausgebildet ist.

9. Endoskop nach einem der obigen Ansprüche, bei dem das Instrumentenrohr (18) und die Bildaufnahmeoptik (19) innerhalb eines Endoskoprohrs (14) angeordnet sind, das in das innere Rohr (6) derart eingesetzt ist, daß zwischen dem inneren Rohr (6) und dem Endoskoprohr (14) der zweite Kanal (15) ausgebildet ist.

10. Endoskop nach Anspruch 9, bei dem das Endoskoprohr (14) einen ovalen Querschnitt aufweist, wobei, im Querschnitt gesehen, die maximale lichte Weite im Bereich des Instrumentenrohrs (18) größer ist als die maximale lichte Weite im Bereich der Bildaufnahmeoptik (19).

11. Endoskop nach einem der obigen Ansprüche, bei dem am proximalen Ende des Instrumentenrohrs (18) ein Dichtsystem (24) angeordnet ist, das das proximale Ende des Instrumentenrohrs (18) unabhängig davon abdichtet, ob in das Instrumentenrohr ein Instrument eingeführt ist oder nicht.

12. Endoskop nach einem der obigen Ansprüche, bei dem das Instrumentenrohr (18) und die Bildaufnahmeoptik (19) miteinander verbunden sind und eine erste Endoskopeinheit (29) bilden, und bei dem eine zweite Endoskopeinheit mit einem weiteren Instrumentenrohr und einer weiteren Bildaufnahmeoptik vorgesehen ist, wobei die Endoskopeinheiten (29) jeweils in das innere Rohr (6) einsetzbar und im eingesetzten Zustand mit dem inneren Rohr (6) verbindbar sind.

## Claims

1. Endoscope comprising a shaft (1) having distal and proximal ends (2, 3), which shaft (1) comprises an outer tube (5) in which an inner tube (6) is inserted such that it is rotatable relative to the outer tube (5) and such that a first channel (7) is formed between the two tubes (5, 6), with image-recording optics (19) being arranged in the inner tube (6) and a second channel (15) being formed therein, and with the image-recording optics (19) being connected with the inner tube (6) in a manner locked against rotation relative to each other, **characterised in that** the inner tube (6) is displaceable relative to the outer tube in the longitudinal direction of the tubes (5, 6) and that an instrument tube (18) is arranged in the inner tube in a manner locked against rotation.

2. Endoscope according to claim 1, wherein both the instrument tube (18) and the image-recording optics (19) are connected with the inner tube (6) such that they are not displaceable relative to the inner tube (6) in the longitudinal direction.

3. Endoscope according to any one of the above claims, wherein the outer tube (5) is open at the distal end (2) and is chamfered, as viewed in a lateral view, relative to the longitudinal direction (L) of the shaft (1).

4. Endoscope according to any one of the above claims, wherein both the instrument tube (18) and the image-recording optics (19) are releasably connected with the inner tube (6).

5. Endoscope according to any one of the above claims, wherein a first shutoff valve (10) is arranged at the proximal end (3) of the shaft (1), said shutoff valve (10) communicating with the first channel (7) and preferably being arranged so as to be rotatable about the longitudinal direction (L) or about an axis parallel to the longitudinal direction (L) and further preferably being connected with the outer tube (5) such that it is not displaceable relative to the outer tube (5) in the longitudinal direction.

6. Endoscope according to any one of the above claims, wherein a second shutoff valve (16) is arranged at the proximal end of the shaft (1), said second shutoff valve (16) communicating with the second channel (15) and preferably being arranged so as to be rotatable about the longitudinal direction (L) or about an axis parallel to the longitudinal direction (L) and further preferably being connected with the inner tube (6) such that it is not displaceable relative to the inner tube (6) in the longitudinal direction.

7. Endoscope according to any one of the above claims, wherein the inner tube (6) has an oval cross-section.

8. Endoscope according to any one of the above claims, wherein the inner tube (6) is open at the distal end and is chamfered, as viewed in a lateral view, relative to the longitudinal direction.

9. Endoscope according to any one of the above claims, wherein the instrument tube (18) and the image-recording optics (19) are arranged within an endoscope tube (14) which is inserted in the inner tube (6) such that the second channel (15) is formed between the inner tube (6) and the endoscope tube (14).

10. Endoscope according to claim 9, wherein the endoscope tube (14) has an oval cross-section, with the maximum clearance in the region of the instrument tube (18) being larger than the maximum clearance in the region of the image-recording optics (19), as viewed in cross-section.

11. Endoscope according to any one of the above claims, wherein a sealing system (24) is arranged at the proximal end of the instrument tube (18), said sealing system (24) sealing the proximal end of the instrument tube (18), whether an instrument is inserted in the instrument tube or not.

12. Endoscope according to any one of the above claims, wherein the instrument tube (18) and the image-recording optics (19) are connected with each other and form a first endoscope unit (29), and wherein a second endoscope unit comprising a further instrument tube and further image-recording optics is provided, said endoscope units (29) each being insertable in the inner tube (6) and being connectable with the inner tube (6) in the inserted condition.

## Revendications

1. Endoscope avec une tige (1) comportant une extrémité distale et une extrémité proximale (2, 3) qui comporte un tube extérieur (5) dans lequel un tube intérieur (6) est inséré de telle manière qu'il est disposé de manière à pouvoir tourner par rapport au tube extérieur (5) et qu'un premier conduit (7) est formé entre les deux tubes (5, 6), une optique de prise de vue (19) étant disposée et un deuxième conduit (15) étant formé dans le tube intérieur (6) et l'optique de prise de vue (19) étant raccordée au tube intérieur (6) fixe en rotation, **caractérisé en ce que** le tube intérieur (6) est déplaçable par rapport au tube extérieur (5) dans la direction longitudinale des tubes (5, 6) et **en ce qu'**un tube d'instrument (18) est disposé dans le tube intérieur (6) de manière à résister à la rotation.

2. Endoscope selon la revendication 1, dans lequel le tube d'instrument (18) et l'optique de prise de vue (19) sont tous deux raccordés au tube intérieur (6) de telle manière qu'ils ne soient pas déplaçables par rapport au tube intérieur (6) dans la direction longitudinale.

3. Endoscope selon l'une quelconque des revendications précédentes, dans lequel le tube extérieur (5) est ouvert à l'extrémité distale (2) et, suivant une vue latérale, il a une configuration chanfreinée par rapport à la direction longitudinale (L) de la tige (1).

4. Endoscope selon l'une quelconque des revendications précédentes, dans lequel le tube d'instrument (18) et l'optique de prise de vue (19) sont tous deux raccordés de manière détachable au tube intérieur (6).

5. Endoscope selon l'une quelconque des revendications précédentes, dans lequel un premier robinet d'arrêt (10) est disposé à l'extrémité proximale (3) de la tige (1), lequel robinet communique avec le premier conduit (7) et est disposé, de préférence, en rotation autour de la direction longitudinale (L) ou d'un axe parallèle à la direction longitudinale (L) et qui, en outre, est raccordé, de préférence, au tube extérieur (5) de telle manière qu'il ne soit pas déplaçable par rapport au tube extérieur (5) dans la direction longitudinale.

6. Endoscope selon l'une quelconque des revendications précédentes, dans lequel un deuxième robinet d'arrêt (16) est disposé à l'extrémité proximale de la tige (1), lequel robinet communique avec le deuxième conduit (15) et est disposé, de préférence, en rotation autour de la direction longitudinale (L) ou d'un axe parallèle à la direction longitudinale (L) et qui, en outre, est raccordé, de préférence, au tube intérieur (6) de telle manière qu'il ne soit pas déplaçable par rapport au tube intérieur (6) dans la direction longitudinale.

7. Endoscope selon l'une quelconque des revendications précédentes, dans lequel le tube intérieur (6) présente une section transversale ovale.

8. Endoscope selon l'une quelconque des revendications précédentes, dans lequel le tube intérieur (6) est ouvert à l'extrémité distale et, suivant une vue latérale, il a une configuration chanfreinée par rapport à la direction longitudinale.

9. Endoscope selon l'une quelconque des revendications précédentes, dans lequel le tube d'instrument (18) et l'optique de prise de vue (19) sont tous deux disposés à l'intérieur d'un tube d'endoscope (14) qui est inséré dans le tube intérieur (6) de manière à former le deuxième conduit (15) entre le tube intérieur (6) et le tube d'endoscope (14).

10. Endoscope selon la revendication 9, dans lequel le tube d'endoscope (14) présente une section transversale ovale, moyennant quoi, vu en coupe transversale, le diamètre intérieur maximal dans la zone du tube d'instrument (18) est plus grand que le diamètre intérieur maximal dans la zone de l'optique de prise de vue (19).

11. Endoscope selon l'une quelconque des revendications précédentes, dans lequel un système d'étanchéité (24) est disposé à l'extrémité proximale du tube d'instrument (18), lequel système étanche l'extrémité proximale du tube d'instrument (18), qu'un instrument soit introduit ou non dans le tube d'instrument.

12. Endoscope selon l'une quelconque des revendications précédentes, dans lequel le tube d'instrument (18) et l'optique de prise de vue (19) sont raccordés l'un à l'autre et forment une première unité d'endoscopie (29) et dans lequel il est prévu une deuxième unité d'endoscopie avec un autre tube d'instrument et une autre optique de prise de vue, les unités d'endoscopie (29) pouvant être insérées chacune dans le tube intérieur (6) et raccordées au tube intérieur (6) à l'état inséré.
